# EUROPÄISCHE PATENTSCHRIFT

(11) **EP 1 816 970 B1**
(45) Veröffentlichungstag und Bekanntmachung des Hinweises auf die Patenterteilung: **08.08.2012**
(21) Anmeldenummer: 05802292.2
(22) Anmeldetag: 28.10.2005
(51) Int. Cl.: A61B 18/14, A61B 17/32

(54) **VORRICHTUNG ZUR RESEKTION UND/ODER ABLATION VON ORGANISCHEM GEWEBE MITTELS HOCHFREQUENZSTROM**
DEVICE FOR THE RESECTION AND/OR ABLATION OR ORGANIC TISSUE BY MEANS OF A HIGH FREQUENCY CURRENT
DISPOSITIF DE RESECTION ET/OU D'ABLATION DE TISSU ORGANIQUE AU MOYEN D'UN COURANT HAUTE FREQUENCE

(30) Priorität: 29.10.2004 FR 0411627
(43) Veröffentlichungstag der Anmeldung: 15.08.2007
(73) Patentinhaber: Hamou, Jacques, 75016 Paris (FR)
(72) Erfinder: Hamou, Jacques, 75016 Paris (FR)
(74) Vertreter: Heuckeroth, Volker
(86) Internationale Anmeldenummer: PCT/EP2005/011579
(87) Internationale Veröffentlichungsnummer: WO 2006/048199

(56) Entgegenhaltungen:
- EP-A- 0 448 857
- WO-A-95/30377
- WO-A-98/24372
- DE-A1- 3 313 325
- FR-A- 2 645 008
- GB-A- 618 528

## Beschreibung

Die Erfindung betrifft eine Vorrichtung zur Resektion und/oder Ablation von organischem Gewebe mittels Hochfrequenzstrom, mit zumindest einer Wendel, wobei die Wendel um die Längsachse der Wendel in Rotation versetzbar ist.

Eine derartige Vorrichtung ist aus WO 98/24372 A1 bekannt.

Die aus WO 98/24372 A1 bekannte Vorrichtung weist einen als Mikromorcellator bezeichneten Kopf zum Schneiden von Gewebe auf. Der Mikromorcellator weist einen Schneidkopf auf, der ein rohrförmiges Element aufweist, in dem eine Klinge angeordnet ist. Ein distaler Rand des rohrförmigen Elements weist eine angeschärfte Abschrägung auf. Die Klinge ist unmittelbar unterhalb des oder bündig mit dem distalen Rand des rohrförmigen Elements angeordnet. Die Klinge besteht vorzugsweise aus einer Windung einer Doppelwendel und ist dazu ausgestaltet, Gewebe zu schneiden, das in das rohrförmige Element hineinragt. Der Schneidkopf ist an einem Antriebsschaft so befestigt, dass die Wendel der Klinge in einer Richtung in Rotation versetzt werden kann, die geeignet ist, um abgetrenntes Gewebe in das Innere des Antriebsschafts zu drücken. Die wendelförmige Klinge kann sich bis zum proximalen Ende des Schafts erstrecken. In diesem Fall wirkt die Wendelstruktur als Archimedesschraube, so dass die Drehung der Wendel abgetrenntes Gewebe nach proximal befördert. Das rohrförmige Element des Schneidkopfs kann aus einem elektrisch leitfähigen Material gefertigt sein und mit HF-Spannung beaufschlagt werden.

Eine Resektion oder Ablation organischer Strukturen wird beispielsweise in der Hysteroskopie zur Entfernung von Fibromen, Polypen und Gewebe des Endometriums und in der Urologie zur Entfernung von prostatischen Adenomen vorgenommen. Derartige Resektionen oder Ablationen organischer Strukturen werden herkömmlicherweise mittels eines Resektionsinstruments durchgeführt, das am distalen Ende eine elektrisch leitende und mit Hochfrequenzstrom beaufschlagbare Metallschlinge aufweist. Zur Durchführung eines Schneidvorganges wird die Schlinge mit monopolarer oder bipolarer Hochfrequenzspannung beaufschlagt und vom operierenden Arzt zum Herbeiführen eines Schnittes zum Abtrennen von Gewebe manuell in Richtung der Längsachse des Resektionsinstruments bewegt, und zwar in einer hin- und hergehenden Bewegung oder nur in einer Richtung. Die mit Hochfrequenzstrom beaufschlagte Schlinge verhält sich dabei wie ein elektrisches Skalpell. Die Schlinge ermöglicht auf diese Weise die Abtrennung von Gewebestücken gleichen Durchmessers. Eine Stillung von Blutungen, die mit dem Abtrennen von Gewebe einhergehen, kann ebenfalls mit derselben Schlinge durchgeführt werden, nachdem das Gewebestück von der Schlinge abgelöst wurde. Die so abgelösten Gewebestücke müssen zu einer anderen Zeit dann auf mechanische Weise aus dem Behandlungsareal entnommen werden.

Ein Nachteil derartiger herkömmlicher Vorrichtungen zur Resektion von Gewebe mittels Hochfrequenzstrom besteht darin, dass auf Grund der Tatsache, dass die abgetrennten Gewebestücke nach dem Abtrennen mittels eines weiteren Instruments, beispielsweise einer Fasszange, aus dem Behandlungsareal entnommen werden müssen, die Dauer des Eingriffs am Patienten erhöht ist. Die erhöhte Dauer des Eingriffs ist durch den erforderlichen mehrfachen Instrumentenwechsel zwischen dem Resektionsinstrument für den Vorgang des Abtrennens von Gewebe und dem Entnahmeinstrument für den Vorgang des Entnehmens des abgetrennten Gewebes bedingt. Ein weiterer Nachteil der herkömmlichen Vorrichtungen ist die Beschränkung ihres Einsatzes auf die bestimmte Indikation, für die sie vorgesehen sind. Des Weiteren ist die Sichtkontrolle bei diesen Vorrichtungen schwierig.

WO 95/30377 A1 offenbart ein chirurgisches Instrument zur Behandlung der Prostata. Das Instrument weist ein Rohr auf, in dem Kanäle für die Beobachtung, Beleuchtung, für die Zuführung von Spülflüssigkeit und für die Abführung von Flüssigkeit und abgeschnittenem Gewebe vorhanden sind. Das Rohr enthält eine motorgetriebene Welle, wobei die Welle an ihrem distalen Ende ein Messer trägt. Über die Welle kann das Messer, das in Form einer Schlinge ausgebildet ist, in Rotation versetzt werden.

FR 2 645 008 offenbart ein chirurgisches Instrument zur Resektion von Gewebe, das am distalen Ende einer in Rotation versetzbaren Welle eine Schlinge aufweist, die am distalen Ende quer von der Welle absteht. Über die Welle kann die Schlinge in Rotation versetzt werden, um Gewebe zu schneiden.

Der Erfindung liegt die Aufgabe zugrunde, eine Vorrichtung der eingangs genannten Art dahingehend weiterzubilden, dass die vorstehend beschriebenen Nachteile vermieden werden, dass insbesondere die Dauer eines Resektionseingriffs verringert werden kann.

Erfindungsgemäß wird diese Aufgabe hinsichtlich der eingangs genannten Vorrichtung dadurch gelöst, dass distal von der zumindest einen Wendel eine Schlinge angeordnet ist, wobei die Wendel und die Schlinge mit Hochfrequenzspannung beaufschlagbar sind, und dass die Anordnung aus Schlinge und Wendel um die Längsachse der Wendel in Rotation versetzbar ist.

Mit der rotierenden distalen Schlinge können vorteilhafterweise Gewebestrukturen abgetrennt werden, die sich frontal vor der Schlinge befinden. Mit der sich an die Schlinge unmittelbar oder mittelbar anschließenden zumindest einen Wendel können dagegen Gewebestrukturen abgetrennt werden, die sich seitlich von der zumindest einen Wendel befinden. Durch die Beaufschlagung der Schlinge und der zumindest einen Wendel mit Hochfrequenzstrom wird das Abtrennen von Gewebe unter der Wirkung des Hochfrequenzstroms noch weiter verbessert. Ebenso können mittels Hochfrequenzstrom jedoch auch Blutungen durch Koagulation gestillt werden, die beim Abtrennen des Gewebes auftreten. Eine weitere Wirkung der rotierenden Wendel besteht nun vorteilhafterweise darin, dass abgetrenntes Gewebe in das Innere der Wendel mitgenommen werden kann, und wobei dieses in das Innere der Wendel eintretende abgetrennte Gewebe durch die Rotation der zumindest einen Wendel in Richtung proximales Ende der Vorrichtung transportiert werden kann. Mit der erfindungsgemäßen Vorrichtung ist es somit ohne Instrumentenwechsel möglich, eine Resektion von organischem Gewebe ununterbrochen durchzuführen und dabei gleichzeitig das abgetrennte Gewebe zu entnehmen und Blutungen zu stillen. Die erfindungsgemäße Vorrichtung ermöglicht es folglich, die Dauer eines Eingriffs gegenüber den herkömmlichen Vorrichtungen zu verringern. Die Vorrichtung eignet sich für monopolare, aber auch für bipolare Ausgestaltungen. In einer bevorzugten Ausgestaltung ist die Wendel als Doppelwendel oder Mehrfachwendel ausgebildet.

"Doppelwendel" oder "Mehrfachwendel" bedeutet hier, dass die Wendel aus zwei oder mehreren schraubenlinienförmigen und vorzugsweise zylindrischen Strukturen aufgebaut ist. Die Ausgestaltung der zumindest einen Wendel als Doppelwendel oder Mehrfachwendel führt zum einen zu einer höheren Verwindungssteifigkeit der Wendel beim Resezieren festeren Gewebes, insbesondere wenn die Wendel in Längsrichtung lang ausgebildet ist, und außerdem kann auf diese Weise die Wirkung der Mitnahme abgetrennten Gewebes nach proximal noch verbessert werden.

Wie erwähnt, ist die zumindest eine Wendel vorzugsweise so ausgebildet, dass sie eine Abtragung seitlich von der Wendel befindlichen Gewebes ermöglicht.

Weiterhin ist die Wendel vorzugsweise so ausgebildet, dass sie einen Transport abgetrennten Gewebes nach proximal ermöglicht.

In einer weiteren bevorzugten Ausgestaltung ist die Wendel teilweise von einem Rohr umgeben und mit diesem zumindest teilweise fest verbunden, wobei ein distaler Abschnitt der Wendel aus einer distalen Öffnung des Rohrs aus dem Rohr herausragt.

Hierbei ist von Vorteil, dass das Rohr eine noch weiter erhöhte Verwindungssteifigkeit der Wendel gewährleistet. Der aus der distalen Öffnung des Rohrs herausragende distale Abschnitt der Wendel ist dann der aktiv schneidende und zumindest der Abschnitt, der mit Hochfrequenzstrom beaufschlagt wird. Die feste Verbindung zwischen Wendel und Rohr kann beispielsweise durch Kleben hergestellt sein.

Dabei ist es bevorzugt, wenn das Rohr mittels eines Motors in Rotation versetzbar ist.

Hierdurch wird ein konstruktiv einfacher Antrieb geschaffen, da der Motor lediglich das Rohr in Rotation versetzen muss, und über die feste Verbindung der Wendel mit dem Rohr wird dann die Wendel ebenfalls in Rotation versetzt.

Alternativ zu der Ausgestaltung, wonach die Wendel zumindest teilweise fest mit dem Rohr verbunden ist, ist es alternativ bevorzugt, wenn die Wendel nicht mit dem Rohr fest verbunden ist, sondern relativ zum Rohr frei drehbar ist.

In diesem Fall ist das Rohr vorzugsweise feststehend, und die Wendel wird mittels des Motors in Rotation versetzt. Ein Vorteil der Ausgestaltung, nach der sich nur die Wendel und die Schlinge dreht, besteht darin, dass die Schlinge und die Wendel, die einem Verschleiß unterworfen sind, einzeln ausgetauscht werden können, während das Rohr, das keinem Verschleiß oder keinem wesentlichen Verschleiß unterliegt, weiter verwendet werden kann.

Weiterhin ist es bevorzugt, wenn sich die Wendel zumindest annähernd über die gesamte Länge des Rohrs erstreckt.

Durch diese Maßnahme ist die Mitnahme- bzw. Transportwirkung der Wendel auf das abgetrennte Gewebe bis zum proximalen Ende des Rohrs zur einfachen Entnahme bzw. zum Auffangen des abgetrennten Gewebes in einen Behälter vorhanden.

Alternativ zu der zuvor genannten Ausgestaltung kann es auch bevorzugt sein, wenn sich die Wendel nur über eine Teillänge des Rohrs erstreckt.

Diese Maßnahme ist insbesondere mit der oben genannten Maßnahme, wonach nur die Wendel in Rotation versetzbar ist, vorteilhaft, da der Austausch der Wendel einfacher zu bewerkstelligen ist.

Als Stromzuführung für die Wendel können dann vorzugsweise Blades verwendet werden, bei einer Doppelwendel entsprechend zwei Blades, die vom proximalseitigen HF-Anschluss bis etwa zum proximalen Ende der Wendel sich durch das Rohr 16 erstrecken und dort mit der Wendel kontaktiert sind. Die Blades können über Schleifkontakte, insbesondere Schleifringe, mit dem HF-Anschluss verbunden sein, da die Blades mit der Wendel mitrotieren müssen. Die Blades können bei einer austauschbaren Ausgestaltung der Wendel im Rohr verbleiben.

Die leichtere Austauschbarkeit der Schlinge und der Wendel ohne Austauschbarkeit des Rohrs hat des Weiteren den Vorteil, dass für verschiedene Anwendungen rasch eine Ersatzschlinge mit Ersatzwendel in die Vorrichtung eingebaut werden können, ohne dass dazu eine größere Zerlegung der Vorrichtung erforderlich ist.

In einer weiteren bevorzugten Ausgestaltung ist der sich im Rohr erstreckende Abschnitt der Wendel elektrisch isoliert. Hierbei ist von Vorteil, dass das im Innern der Wendel durch das Rohr transportierte abgetrennte Gewebe nicht an der Wendel auf Grund von Erwärmung anhaftet.

Ebenso ist bevorzugt, wenn das Rohr elektrisch isoliert ist.

Hierdurch wird eine Gefahr für den bedienenden Arzt vermieden.

Dagegen ist der aus dem Rohr herausragende Abschnitt der Wendel vorzugsweise nicht elektrisch isoliert.

In einer weiteren bevorzugten Ausgestaltung ist der aus dem Rohr herausragende Abschnitt der Wendel aus einem steifen Draht gebildet oder als Schneidklinge mit nach innen gerichteter Schneide ausgebildet.

Insbesondere die Ausgestaltung der Wendel als Schneidklinge mit nach innen gerichteter Schneide in ihrem aus dem Rohr herausragenden Abschnitt ist geeignet, das abgetrennte Gewebe weiter zu morcellieren und den Transport des abgetrennten Gewebes in das Innere der zumindest einen Wendel zu begünstigen.

In einer weiteren bevorzugten Ausgestaltung weist der sich im Rohr erstreckende Abschnitt der Wendel, der vorzugsweise eine variable Breite aufweist, eine nach innen gerichtete Schneide auf.

Die nach innen gerichtete Schneide hat den Vorteil, dass der Transport von in das Rohr eintretenden bzw. eingetretenen abgetrennten Gewebestücken nach proximal weiter begünstigt wird.

In weiteren bevorzugten Ausgestaltungen kann die distale Öffnung des Rohrs einen stumpfen oder schneidenden Rand aufweisen.

Das Rohr weist vorzugsweise einen Durchmesser im Bereich von etwa 3 bis etwa 6,5 mm auf, wobei jedoch auch außerhalb dieses Bereiches liegende Durchmesser je nach Anwendung in Betracht gezogen werden können.

Die Schlinge am distalen Ende der Vorrichtung ist vorzugsweise halbkreisförmig ausgebildet, und weist vorzugsweise einen Durchmesser von 4 bis 7 mm auf.

Die Schlinge ist weiterhin vorzugsweise aus einem steifen elektrisch leitenden Draht gebildet oder als Schneidklinge ausgebildet, wobei im letzteren Fall die Schneide nach innen gerichtet ist.

Weiterhin ist es bevorzugt, wenn die Ebene der Schlinge gegenüber der Längsachse geneigt, vorzugsweise geringfügig geneigt ist, derart, dass von der Schlinge abgetrenntes Gewebe zum Inneren der Wendel hin orientiert wird.

Hierbei ist von Vorteil, dass das von der Schlinge frontal abgetrennte Gewebe leichter in das Innere der Wendel zwecks Transport nach proximal eintreten kann.

In einer weiteren bevorzugten Ausgestaltung ist die Drehgeschwindigkeit der zumindest einen Wendel und der Schlinge in einem Bereich von 0 bis 1000 Umdrehungen pro Minute, vorzugsweise stufenlos, einstellbar.

Hierbei ist von Vorteil, dass die Drehgeschwindigkeit und damit die Schneidwirkung an die Festigkeit des zu resezierenden Gewebes angepasst werden kann.

Es kann ebenso vorzugsweise vorgesehen sein, dass feste Drehgeschwindigkeiten voreingestellt sind, beispielsweise von 20 Umdrehungen pro Minute, 300 und 800 Umdrehungen pro Minute, die dann beispielsweise durch einen entsprechenden Betätigungsknopf rasch eingestellt werden können.

Ebenso ist es alternativ oder zusätzlich bevorzugt, wenn eine Feinregulierung, in bspw. zwei Bereichen, bspw. in einem Bereich von 0 bis 20 Umdrehungen pro Minute und in einem Bereich von 300 bis 800 Umdrehungen pro Minute vorgesehen ist.

Zur Einstellung bzw. Steuerung der Drehgeschwindigkeit ist vorzugsweise ein an einem proximal an der Vorrichtung vorhandenen Handgriff angeordneter Betätigungsknopf oder ein Fußpedal vorgesehen.

Der am Handgriff angeordnete Betätigungsknopf ist vorzugsweise so positioniert, dass er mit derselben Hand betätigbar ist, mit der die Vorrichtung in der Hand gehalten wird.

In einer weiteren bevorzugten Ausgestaltung wird der Hochfrequenzstrom von einem Hochfrequenzstromgenerator bereitgestellt und ist zum Schneiden und Koagulieren ausgelegt, wobei der Hochfrequenzstrom, vorzugsweise durch ein Fußpedal, einschaltbar ist.

Die erfindungsgemäße Vorrichtung ist vorzugsweise dazu ausgelegt, sowohl unter der Wirkung von Hochfrequenzstrom zu schneiden als auch unter der Wirkung von Hochfrequenzstrom zu koagulieren, wozu ggf. die Parameter des Hochfrequenzstroms wie Amplitude, Frequenz und ggf. eine Amplitudenmodulation, geeigneterweise ausgelegt und eingestellt sein müssen. Der bedienende Arzt kann selbst die Dauer und den Einsatzzeitpunkt des Hochfrequenzstroms durch Ein- und Ausschalten bestimmen.

In einer weiteren bevorzugten Ausgestaltung ist ein proximales Ende des Innenraums der Wendel, beispielsweise über einen Schlauch, mit einem Auffangbehälter, beispielsweise einer Flasche, zur Aufnahme von Gewebestücken verbunden.

Hierbei ist von Vorteil, dass die über die zumindest eine Wendel von distal nach proximal transportierten Gewebestücke sogleich in einen Behälter abgeführt werden, ohne dass hierzu eine weitere Manipulation erforderlich ist.

In einer weiteren bevorzugten Ausgestaltung ist, beispielsweise mittels einer Pumpe, an den Innenraum der Wendel bzw. des Rohrs ein vorzugsweise einstellbarer Unterdruck anlegbar.

Hierbei ist von Vorteil, dass im Fall, dass der Mitnahmeeffekt der rotierenden Wendel zum Abtransport des abgetrennten Gewebes von distal nach proximal nicht oder nicht vollständig ausreicht, der angelegte Unterdruck die Entnahme der abgetrennten Gewebestücke zumindest unterstützt und eine vollständige Entnahme der abgetrennten Gewebestücke aus dem Körper gewährleistet.

In einer weiteren bevorzugten Ausgestaltung ist ein Außenschaft vorgesehen, um die Anordnung aus dem Rohr und der Wendel und der Schlinge in dem Außenschaft aufzunehmen, wobei die Schlinge und der aus dem Rohr herausragende Abschnitt der Wendel aus dem Außenschaft herausragen.

Hierbei ist von Vorteil, dass sich die Anordnung aus dem Rohr, der Wendel und der Schlinge nach Aufnahme in den Außenschaft ggf. einfacher durch einen Trokar oder einen natürlichen Zugang in das Operationsgebiet einführen lässt und dass die Vorrichtung mit zusätzlichen Funktionen wie Sichtkontrolle, Spülen und dgl. versehen werden kann.

Dabei ist es weiterhin bevorzugt, wenn der Außenschaft zusätzlich zur Aufnahme eines Endoskops ausgelegt ist.

Mit dem Endoskop kann vorteilhafterweise der Vorgang der Resektion im Operationsgebiet unter Sicht kontrolliert werden. Das Endoskop hat vorzugsweise eine Schrägvorausblick-Optik, beispielsweise eine 30°-Optik. Anstelle eines klassischen Endoskops mit einem Bildübertragungssystem aus Linsen kann auch ein Endoskop mit Bildübertragung durch Lichtfasern oder ein Videoendoskop mit distalem Bildsensor verwendet werden.

Des Weiteren ist es bevorzugt, wenn der Außenschaft zumindest eine Leitung zum Zuführen von Spülflüssigkeit in das Operationsgebiet und/oder zumindest eine Leitung zum Absaugen von Flüssigkeit aus dem Operationsgebiet aufweist.

Durch Spülen kann Blut ausgespült werden, wodurch eine gute Sichtkontrolle, beispielsweise durch das zuvor erwähnte Endoskop, aufrecht erhalten werden kann.

Das Zuleiten von Spülflüssigkeit erfolgt vorzugsweise unter geringem Druck. Die Absaugung der Spülflüssigkeit kann durch den verbleibenden Raum innerhalb des Außenschafts nach proximal erfolgen, wobei im distalen Bereich des Außenschafts eine Mehrzahl von Saugöffnungen vorhanden sein können, durch die Flüssigkeit in den Außenschaft hinein angesaugt werden kann.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

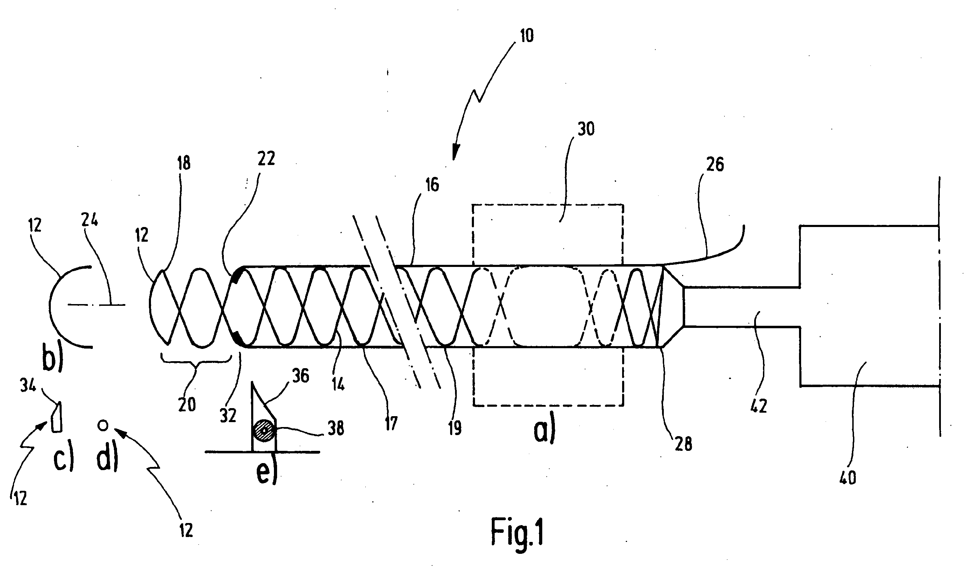

Ausführungsbeispiele der Erfindung sind in der Zeichnung dargestellt und werden mit Bezug auf diese hiernach näher beschrieben. Es zeigen:
- Fig. la) bis e): eine Vorrichtung zur Resektion und/oder Ablation von organischem Gewebe mittels Hochfrequenzstrom, wobei Fig. la) eine schematische Seitenansicht der Vorrichtung und Fig. 1b) bis e) Einzelheiten der Vorrichtung in Alleinstellung und teilweise in vergrößertem Maßstab zeigen;
- Fig. 2: schematisch eine gegenüber Fig. 1 modifizierte Vorrichtung zur Resektion und/oder Ablation von organischem Gewebe mittels Hochfrequenzstrom in Seitenansicht;
- Fig. 3: einen distalen Abschnitt der Vorrichtung in Fig. 2 im Längsschnitt und vergrößertem Maßstab; und
- Fig. 4a) bis e): weitere Einzelheiten der Vorrichtung in Fig. 2 und 3 in verschiedenen Ausführungsvarianten, wobei Fig. 4a) eine Teilseitenansicht und Fig. 4b) bis e) Vorderansichten der Vorrichtung zeigen.

In Fig. la) ist schematisch eine mit dem allgemeinen Bezugszeichen 10 versehene Vorrichtung zum Resezieren von organischem Gewebe mittels Hochfrequenzstrom dargestellt.

Die Vorrichtung 10 kann beispielsweise in der Hysteroskopie zur Entfernung von Fibromen, Polypen und Endometriumsgewebe und beispielsweise in der Urologie zur Entfernung prostatischer Adenome verwendet werden.

Am äußeren distalen Ende weist die Vorrichtung 10 eine mit Hochfrequenzstrom beaufschlagbare Schlinge 12 auf. An die Schlinge 12 schließt sich im gezeigten Ausführungsbeispiel unmittelbar eine als Doppelwendel ausgebildete Wendel 14 an, die teilweise in einem Rohr 16 angeordnet ist.

Ein distales Ende 18 der Wendel 14 ist mit der distalen Schlinge 12 verbunden. Ein distaler Abschnitt 20 der Wendel 14 ragt aus einer distalen Öffnung 22 des Rohrs 16 heraus. Der übrige Abschnitt der Wendel 14, der sich in das Rohr 16 hinein erstreckt, ist mit dem Rohr 16 zumindest teilweise fest verbunden, beispielsweise durch Kleben.

Anstelle einer zumindest teilweise festen Verbindung der Wendel 14 mit dem Rohr 16 kann auch eine Ausgestaltung in Betracht gezogen werden, bei der die Wendel 14 nicht mit dem Rohr 16 verbunden ist, so dass nur die Wendel 14 in Rotation versetzbar ist, während das Rohr 16 feststehend ausgebildet ist.

Zumindest der aus dem Rohr 16 herausragende distale Abschnitt 20 der Wendel 14 ist ebenfalls mit Hochfrequenzspannung beaufschlagbar, und zwar mit derselben Hochfrequenzspannung, mit der auch die distale Schlinge 12 beaufschlagbar ist. Die Schlinge 12 und der distale Abschnitt 20 der Wendel 14 sind entsprechend stromleitend ausgebildet. Der sich durch das Rohr 16 erstreckende Abschnitt der Wendel 14 kann entweder nicht stromleitend ausgebildet sein oder eine entsprechende Isolierung aufweisen, so dass der sich in das Rohr 16 erstreckende Abschnitt der Wendel 14 gegen den distalen Abschnitt 20 elektrisch isoliert ist.

In Fig. la) ist mit dem Bezugszeichen 32 die elektrische Isolierung des sich in das Rohr 16 erstreckenden Abschnitts der Wendel 14 gegenüber dem distalen Abschnitt 20 der Wendel 14 angedeutet.

Die Schlinge 12 ist gemäß Fig. 1b) in Form eines Halbkreises ausgebildet, wobei die Ebene der Schlinge 12 gegenüber einer Längsachse 24 geneigt ist, d.h. mit der Längsachse 24 einen Winkel bildet, oder mit anderen Worten nicht in der Zeichenebene in Fig. la) liegt.

Das Rohr 16 ist ebenfalls elektrisch isoliert. Das Rohr 16 kann anstatt aus Metall auch aus einem vorzugsweise harten Kunststoff ausgebildet sein, in dessen Innenwand der sich in das Rohr 16 erstreckende Abschnitt der Wendel 14 auch eingearbeitet sein kann.

Der distale Abschnitt 20 und damit die Schlinge 12 sind über eine durch das Rohr 16 hindurchgehende elektrische Zuleitung 26, beispielsweise einen mit Isolation versehenen Draht, kontaktiert, wobei die elektrische Zuleitung 26 entsprechend mit einem nicht dargestellten Hochfrequenzstromgenerator verbunden ist oder verbunden werden kann.

Im Fall, dass nur die Wendel 14 in Rotation versetzbar ist, und nicht jedoch das Rohr 16, dient die elektrische Zuleitung 26, die dann bspw. in Form von Blades, d.h. langerstreckten elektrisch leitenden Stangen ausgebildet ist, an der Vorrichtung gehalten und elektrisch kontaktiert.

Die Wendel 14 erstreckt sich im Wesentlichen über die gesamte Länge des Rohrs 16, d.h. reicht bis zu dessen proximalen Ende 28. Die Wendel 14 kann aber auch vor dem proximalen Ende des Rohrs 16 enden, d.h. sich nur über eine Teillänge des Rohrs 16 erstrecken, bspw. bis zu einer Stelle 17 oder 19 in Fig. 1a). Diese Teillänge kann bspw. 4 cm betragen. Der Abschnitt 20 der Wendel 14, der aus dem Rohr 16 herausragt, kann bspw. eine Länge von 4 bis 6 mm haben.

Die Wendel 14 und die Schlinge 12 sind zusammen um die Längsachse 24 der Wendel 14 in Rotation versetzbar. Dazu ist ein Motor 30 vorgesehen, der in dem Ausführungsbeispiel gemäß Fig. 1a) koaxial zur Längsachse 24 bzw. zu dem Rohr 16 angeordnet ist und das Rohr 16 selbst in Rotation versetzt. Über die Rotation des Rohres 16 wird die damit fest verbundene Wendel 14 und die damit fest verbundene Schlinge 12 in Rotation versetzt.

Der Motor 30 ist ein Elektromotor und kann als Hohlwellenmotor ausgebildet sein.

Die Schlinge 12 und der distale Abschnitt 20 der Wendel 14 sind elektrisch nicht isoliert. Wenn das Rohr 16 und damit die Wendel 14 und die Schlinge 12 in Rotation versetzt werden, können mit der Schlinge 12 frontale, d.h. distalseitige organische Strukturen unter der Wirkung von Hochfrequenzstrom geschnitten werden, während mit dem distalen Abschnitt 20 der Wendel 14 seitlich befindliche Gewebestrukturen unter der Wirkung von Hochfrequenzstrom geschnitten werden können. Ebenso kann mit der Schlinge 12 und dem distalen Abschnitt 20 der Wendel 14 koaguliert werden, um eine Blutstillung zu erreichen.

Die Schrägstellung der distalen Schlinge 12 bewirkt bei der Rotation der Schlinge 12, dass das von der Schlinge 12 abzutrennendes Gewebe beim Schneidvorgang bereits so orientiert wird, dass es in das Innere der Wendel 14, d.h. in den zylindrischen Innenraum der Wendel 14 bzw. des Rohrs 16 eintreten kann. Um diesen Effekt noch zu verstärken, kann die Schlinge 12 im Querschnitt gemäß Fig. 1c) als Schneidklinge ausgebildet sein, wobei eine Schneide 34 in das Innere der Wendel 14 zeigend ausgerichtet ist.

Die Schlinge 12 kann jedoch auch aus einem steifen Draht, beispielsweise Stahldraht, gebildet sein, wie in Fig. 1d) veranschaulicht ist, die entsprechend einen runden Querschnitt der Schlinge 12 zeigt. Der Durchmesser des Drahtes kann für Schneiden 1/4 mm, für Koagulation 1 mm betragen, je nach Anwendung. Die Wahl des Durchmessers wird davon abhängen, ob vorwiegend geschnitten oder koaguliert werden soll.

Die Wendel 14 ist in dem gezeigten Ausführungsbeispiel aus zwei in Längsrichtung um die halbe Ganghöhe versetzten schraubenlinienförmigen Windungen aufgebaut und insgesamt von zylindrischer Form. Die Wendel 14 dient insbesondere in ihrem Abschnitt, der sich in das Rohr 16 erstreckt, mittels der Schlinge 12 und dem distalen Abschnitt 20 abgetrenntes Gewebe, das zunächst in das Innere der Wendel 14, eingetreten ist in Richtung proximales Ende des Rohrs 16 zu transportieren, so dass das abgetrennte Gewebe ohne zusätzliche manuelle Eingriffe aus dem Behandlungsareal entnommen werden kann.

Der distale Abschnitt 20 der Wendel 14, der aus dem Rohr 16 herausragt, kann aus einem steifen Draht, insbesondere Stahldraht, gebildet sein, oder als Schneidklinge mit in das Innere der Wendel 14 gerichteter Schneide 36 ausgebildet sein, wie in Fig. 1e) dargestellt ist. Die Ausgestaltung als Schneidklinge mit nach innen gerichteter Schneide 36 begünstigt die Morcellierung des abgetrennten Gewebes und den Eintritt des abgetrennten Gewebes in das Innere der Wendel 14.

Der sich in das Rohr 16 erstreckende Abschnitt der Wendel 14 kann ebenfalls eine in das Innere der Wendel 14 gerichtete Schneide aufweisen, um die resezierten bzw. abgetrennten Gewebestücke, die in das Rohr 16 eintreten, besser in Richtung proximales Ende 28 des Rohrs abzuführen. Wie bereits zuvor erwähnt, ist jedoch der sich durch das Rohr 16 erstreckende Abschnitt der Wendel 14 vorzugsweise elektrisch isoliert, wie in Fig. 1e) mit 38 angedeutet ist. Ferner kann der sich in das Rohr 16 erstreckende Abschnitt der Wendel 14 von variabler Breite bzw. Stärke der Wendel 14 sein.

Der Rand der distalen Öffnung 22 des Rohrs 16 kann selbst scharf und damit schneidend oder stumpf sein.

Der Durchmesser des Rohrs 16 liegt beispielsweise in einem Bereich von etwa 3 bis etwa 6,5 mm, wobei die Wahl des Durchmessers des Rohrs 16 und damit auch der Wendel 14 von der Größe der zu resezierenden organischen Strukturen abhängig sein kann. Die Länge des Rohrs 16 beträgt bspw. 10 bis 30 cm.

Die Drehgeschwindigkeit des Motors 30 und damit der Wendel 14 und der Schlinge 12 ist einstellbar in einem Bereich von 0 bis etwa 1000 Umdrehungen pro Minute, wobei diese Einstellbarkeit vorzugsweise stufenlos ist. Die Drehgeschwindigkeit wird entsprechend der Beschaffenheit des abzutrennenden Gewebes eingestellt. Ein Betätigungselement zum Einstellen der Drehgeschwindigkeit wird später noch mit Bezug auf die weiteren Figuren beschrieben.

Ferner kann die Drehgeschwindigkeit des Motors 30 auch auf feste Drehgeschwindigkeiten einstellbar sein, beispielsweise eine Drehgeschwindigkeit von etwa 20 Umdrehungen pro Minute und eine Drehgeschwindigkeit von etwa 300 Umdrehungen pro Minute und eine weitere bei etwa 800 Umdrehungen pro Minute.

Der von dem oben erwähnten Hochfrequenzstromgenerator bereitgestellte Hochfrequenzstrom, der das Schneiden von Gewebe und die Koagulation ermöglicht, wird über die elektrische Zuleitung 26, die elektrisch isoliert ist, auf die Wendel 14 und die Schlinge 12 übertragen, wobei der Hochfrequenzstrom entsprechend zum Schneiden und/oder Koagulieren ausgelegt ist. In nicht dargestellter Weise ist zum Einschalten bzw. zur Regulierung der Parameter des Hochfrequenzstroms ein Fußpedal vorgesehen. Die Vorrichtung 10 eignet sich für monopolaren Hochfrequenzstrom, kann jedoch auch bei entsprechender Modifikation für bipolare Anwendungen ausgelegt werden.

Das proximale Ende 28 des Rohrs 16 ist ferner mit einem Auffangbehälter 40, beispielsweise einer Flasche, verbunden bzw. verbindbar, wobei die Verbindung beispielsweise über einen biegsamen Schlauch 42 bewerkstelligt wird. Durch das Rohr 16 nach proximal transportiertes abgetrenntes Gewebe wird in dem Auffangbehälter 40 gesammelt.

Die Entnahme abgetrennten Gewebes durch das Rohr 16 hindurch kann weiter verbessert werden, indem an das Innere der Wendel 14 bzw. an das Innere des Rohrs 16 ein bis zu dem Auffangbehälter 40 reichender Unterdruck, beispielsweise mittels einer Pumpe, angelegt wird, wobei der Unterdruck bzw. die Saugleistung variabel einstellbar ist, beispielsweise in einem Bereich von 0 bis -1 bar. Als Pumpe kann bspw. ein Gerät ENDOMAT® verwendet werden

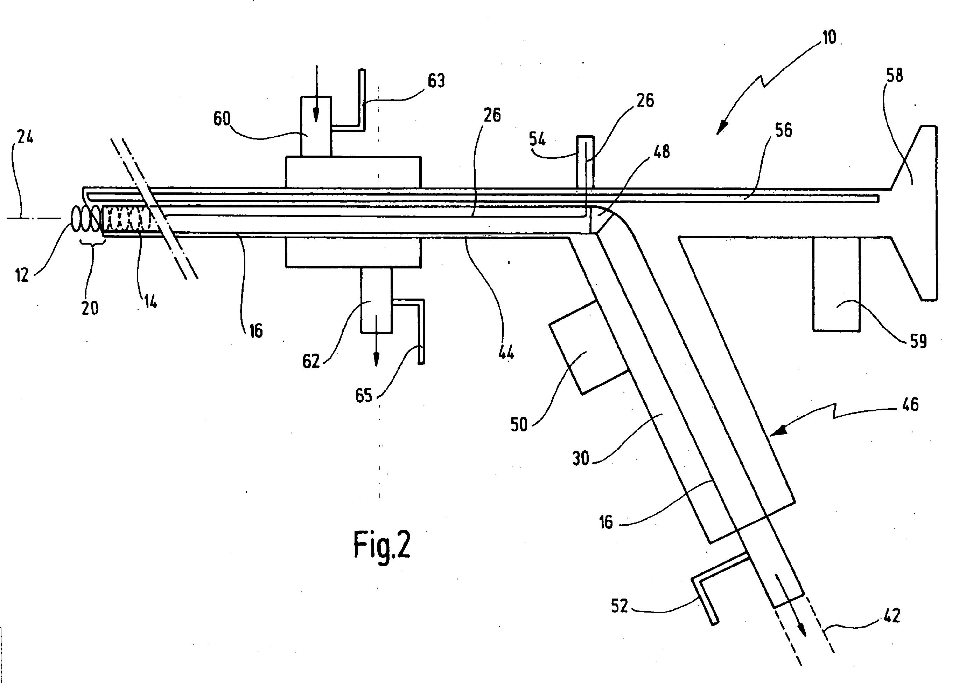

Mit Bezug auf Fig. 2 bis 4 werden nun weitere Ausgestaltungen der Vorrichtung 10 bzw. deren Erweiterungsmöglichkeiten beschrieben.

In Fig. 2 bis 4 bezeichnen gleiche Bezugszeichen wie in Fig. 1 gleiche oder hinsichtlich ihrer Funktion gleiche Teile der Vorrichtung 10.

Gemäß Fig. 2 bis 4 ist die Anordnung aus Rohr 16, Wendel 14 und Schlinge 12 in einem Außenschaft 44 aufgenommen, derart, dass das Rohr 16 und damit die Wendel 14 und die Schlinge 12 in dem Außenschaft 44 in Rotation versetzbar sind.

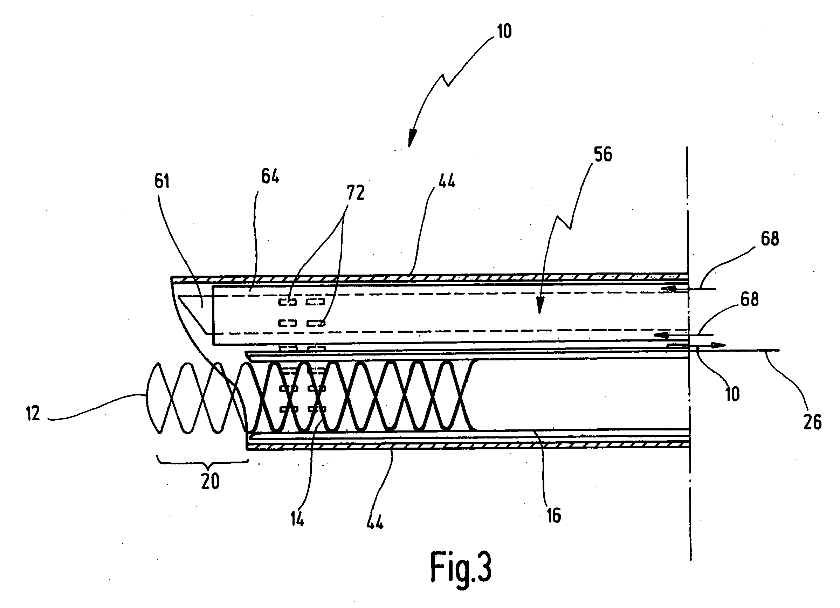

Wie aus Fig. 3 hervorgeht, ist die Anordnung aus Rohr 16, Wendel 14, Schlinge 12 derart getroffen, dass der distale Abschnitt 20, also der zum Schneiden aktive Abschnitt der Wendel 14 und die Schlinge 14 aus dem Außenschaft 44 distal herausragen. In Fig. 3 ist der isolierte, sich in das Rohr 16 erstreckende Abschnitt der Wendel 14 mit dickeren Linien dargestellt.

Bei der Vorrichtung, gemäß Fig. 1 und der Vorrichtung gemäß Fig. 2, 3 können das Rohr 16, die Wendel 14 und die Schlinge 12 insbesondere als Baueinheit austauschbar ausgestaltet sein, insbesondere als Einwegteil. Dabei können unterschiedliche Wendelbreiten für die Wendel 14 vorgehalten werden, die jeweils mit dem Außenschaft 44 verwendbar sind. Bei einer Ausgestaltung als Einwegteil bietet sich insbesondere eine Ausgestaltung des Rohrs 16 als Kunststoffteil an.

Bei der Ausgestaltung, wonach sich nur die Wendel 14 dreht, nicht aber das Rohr 16, ist die Wendel 14 mit der Schlinge 12 einzeln austauschbar.

Es können Wendeln 14 unterschiedlicher Durchmesser und unterschiedlicher Ganghöhen der Windungen und unterschiedlicher Querschnittsgeometrien der Wendel 14 bereitgehalten werden.

An dem Außenschaft 44 ist ein Handgriff 46 vorhanden, der im Bereich des proximalen Endes der Vorrichtung 10 schräg zur Längsachse 24 ausgerichtet ist.

Der Außenschaft 44 kann beispielsweise einen Durchmesser von 7 bis 10 mm aufweisen, ohne auch hierauf beschränkt zu sein.

Bei dem Ausführungsbeispiel in Fig. 2 reicht das Rohr 16 bis in den Handgriff 46 hinein, wobei der Motor 30 ebenfalls im Handgriff 46 aufgenommen ist. Bei dieser Ausgestaltung ist das Rohr 16 entsprechend mit einem Gelenk 48 versehen, das die Rotation des Abschnittes des Rohrs 16 im Handgriff 46 auf den Abschnitt des Rohrs 16 überträgt, der sich parallel zur Längsachse 24 erstreckt. Das Gelenk 48 kann als Kegelradgetriebe, Kardangelenk oder als flexible Ausführung des Rohrs 16 in diesem Bereich realisiert sein, um Beispiele zu nennen.

Der Motor 30 kann anstelle wie in Fig. la) und anstelle wie in Fig. 2 auch außerhalb der Vorrichtung 10 angeordnet sein. Ein derartiger externer Motor kann dann über eine Welle mit der Vorrichtung 10 verbunden werden, um das Rohr 16 anzutreiben.

An dem Handgriff 46 ist ferner ein Handknopf, insbesondere ein niederdrückbarer Schalter 50 angeordnet, der beispielsweise mit dem Zeigefinger der Hand, die den Handgriff 46 hält, betätigt werden kann, um die Drehgeschwindigkeit des Rohrs 16 und damit der Wendel 14 und der Schlinge 12 stufenlos einzustellen bzw. zu steuern. Des Weiteren ist am Handgriff ein Hahn, Ventil oder dgl. 52 angeordnet, mit dem der an das Innere des Rohrs 16 angelegte Unterdruck bzw. die angelegte Saugleistung zur Unterstützung des Transports abgetrennten Gewebes in den Auffangbehälter 40 (vgl. Fig_{.} 1) eingestellt werden kann. Der Hahn, Ventil oder dgl. 52 kann auch näher zu dem Schalter 50 positioniert sein, so dass eine Betätigung mit dem Mittelfinger derselben Hand, die den Handgriff 46 hält, auf ergonomische Weise ermöglicht wird.

Die Saugleistung durch das Rohr 16 sollte feinfühlig steuerbar sein, damit die davon abhängige Größe des Arbeitsraumes kontrolliert werden kann. Eine zu hohe Saugleistung kann nämlich zu einer unerwünschten Kollabierung des Arbeitsraumes führen.

An dem Außenschaft 44 ist ferner ein Anschluss 54 für das HF-Kabel angeordnet, mit dem die Vorrichtung 10 mit dem Hochfrequenzstromgenerator verbunden werden kann. Da die elektrische Zuleitung zu der Wendel 14 mit der Wendel 14 mitrotieren muss, ist die Zuleitung mit einem Schleifkontakt, bspw. einem Schleifring versehen, um die Stromübertragung vom feststehenden Anschluss 54 auf die Wendel 14 zu gewährleisten.

Der Außenschaft 44 ist des Weiteren zur Aufnahme eines Endoskops 56 ausgelegt, das in Fig. 2 schematisch am proximalen Ende mit einem Okular 58 veranschaulicht ist. Anstelle des Okulars 58 kann jedoch auch eine mit dem Endoskop 56 verbundene Videokamera vorgesehen sein. Ferner ist ein Lichtleiteranschluss 59 zum Anschließen eines Lichtkabels für Beleuchtungslicht vorgesehen.

Insbesondere bei der Anordnung gemäß Fig. la), bei der der Motor 30 koaxial zur Längsachse 24 des Rohrs 16 angeordnet ist, anstatt wie in Fig. 2 seitlich dazu, kann auch ein Endoskop verwendet werden, das am distalen Ende einen Bildsensor aufweist, so dass diesbezüglich keine Schwierigkeiten bei der Vorbeiführung des Endoskops an dem Motor 30 entstehen.

Ein distales Ende 61 des Endoskops 56 (s. Fig. 3) ist etwa auf Höhe des distalen Abschnitts 20 der Wendel 14 bzw. der Schlinge 12 positioniert und weist vorzugsweise eine Schrägvorausblick-Optik auf, beispielsweise eine 30°-Optik.

Mit dem Endoskop 56 wird eine permanente Sichtkontrolle des Schneid- und Koagulationsvorgangs im Behandlungsareal ermöglicht.

Der Durchmesser des Endoskopschafts bzw. der Endoskopoptik kann beispielsweise 3 mm betragen.

Der Außenschaft 44 dient weiterhin zum Zuführen einer Spülflüssigkeit in das Behandlungsgebiet sowie zum Abführen von Flüssigkeit aus dem Behandlungsgebiet. Dazu ist am Außenschaft 44 ein Spülanschluss 60 mit Hahn oder Ventil oder dgl. 63 und ein Sauganschluss 62 mit Hahn oder Ventil oder dgl. 65 vorgesehen, wobei diese beiden Anschlüsse mit einer geeigneten Saug- und Spülquelle (nicht dargestellt) verbunden sind.

Die Bedienelemente 63 und 65 können auch am Handgriff 46 positioniert sein, so dass sie bspw. mit dem Daumen der Hand betätigt werden können, die den Handgriff 46 hält. Die Vorrichtung 10 ist dann einschließlich der Bedienelemente 50 und 52 für eine Einhandbedienung geeignet.

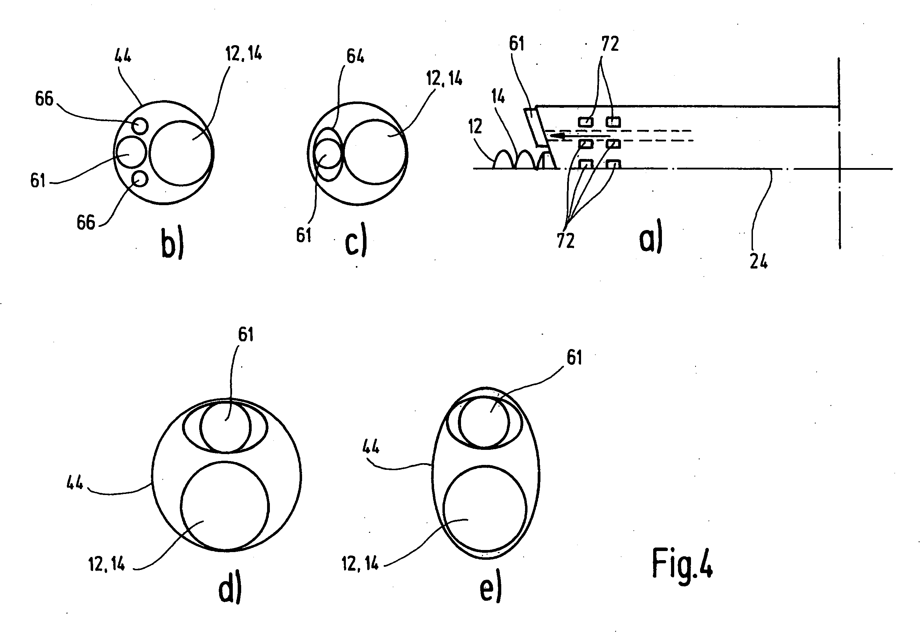

Die permanente Spülung des Operationsgebietes und des Bereichs des distalen Abschnitts 20 der Wendel 14 und der Schlinge 12 wird durch einen ununterbrochenen Fluss einer nichtleitenden Flüssigkeit, wie beispielsweise 1,5 %iges GLYCOCOLLE, ermöglicht, die unter geringem Druck in das Operationsgebiet injiziert wird. Die Zuleitung der Spülflüssigkeit durch den Außenschaft 44 kann beispielsweise durch ein Innenrohr 64 erfolgen, durch das auch das Endoskop 56 hindurchgeführt ist, wobei der verbleibende Ringraum zwischen Endoskop 56 und der Innenwand des Innenrohrs 64 dann der Zuleitung der Spülflüssigkeit dient (s. auch Fig. 4c), d) und e)).

Alternativ können vom Spülanschluss 60 auch dünne Schläuche 66 zum distalen Ende des Außenschafts 44 führen.

Die Absaugung von Flüssigkeiten aus dem Behandlungsgebiet erfolgt über den Sauganschluss 62, wobei die Flüssigkeit durch den verbleibenden Raum im Innern des Außenschafts 44 zwischen dem Rohr 16 und dem Endoskop 56 bzw. dem Rohr 64 abgesaugt wird. In Fig. 3 ist mit Pfeilen 68 die Spülrichtung und mit einem Pfeil 70 die Saugrichtung angedeutet.

In einem distalen Abschnitt des Außenschafts 44 sind ferner Öffnungen bzw. Durchbrechungen 72, beispielsweise auf einer Länge von 2 cm des Außenschafts 44, vorzugsweise vollumfänglich, vorgesehen, durch die Flüssigkeit in den Außenschaft 44 hinein eingesaugt werden kann.

Gemäß Fig. 4e) kann der Außenschaft 44 auch anstatt im Querschnitt rund oval ausgebildet sein.

Die Vorrichtung 10 ermöglicht das Abtrennen frontaler oder seitlicher Gewebestrukturen und ebenso die Koagulation und Entnahme abgetrennten Gewebes aus dem Operationsgebiet heraus. Es sind keine mehrfachen Unterbrechungen oder Instrumentenwechsel erforderlich, um die Blutstillung oder Entnahme der abgetrennten Gewebestücke zu ermöglichen. Während der Resektion kann auf den nicht zu resezierenden Teil des Gewebes ein leichter Zug ausgeübt werden, um die Basis dieser Gewebestruktur besser freizulegen oder sogar abtrennen zu können, um ein unerwünschtes Anschneiden unbeteiligter Strukturen zu vermeiden.

Bei einer Anwendung der Vorrichtung 10 kann bspw. die Resektion eines Myoms bei einer Drehzahl der Schlinge 12 und der Wendel 14 von 0 bis 10 Umdrehungen pro Minute leicht eingeleitet werden, bis das Gewebe in das Rohr 16 eingeführt ist, und zwar bei geschlossenem Hahn bzw. Ventil 52. Währenddessen wird über den Spülanschluss 63 Spülflüssigkeit in das Operationsgebiet eingeleitet und über den Sauganschluss 62 abgeführt, wobei die Bilanz zwischen Zuführung der Spülflüssigkeit und Absaugung von Flüssigkeit derart ist, dass ein leichter Überdruck zur Distension im Operationsareal vorhanden ist. Sobald abgetrenntes Gewebe im Rohr 16 ist, wird dann der Hahn bzw. das Ventil 52 zur Absaugung des abgetrennten Gewebes aus dem Rohr 16 geöffnet und dann wieder geschlossen.

Bei abgeschaltetem Zustand, d.h. bei Drehzahl 0 oder nahe 0 kann mit der Schlinge bzw. dem distalen Abschnitt 20 der Wendel 14 koaguliert werden.

Die schnellere Geschwindigkeit von 50 bis 300 Umdrehungen pro Minute bei partiell geöffnetem Hahn oder Ventil 52 ermöglicht es, schnell alle brüchigen und weichen Gewebe (bspw. des Endometriums) abzusaugen. Die schnelle Geschwindigkeit wird hauptsächlich für endometriale Resektionen und die Resektion bestimmter Polypen eingesetzt.

Die Gewebeabsaugung erfolgt vorzugsweise mit dem Gerät ENDOMAT®. Der Schalter 50 zur Regulierung der Drehgeschwindigkeit der Wendel 14 und der Schlinge 12 ist dazu ausgelegt, die Geschwindigkeit hochpräzise und progressiv zu steuern.

Nach Distension des Operationsgebietes und Einführung der Vorrichtung 10 wird die zu resezierende bzw. abzutragende Gewebestruktur (Myom, Polyp oder Prostataadenom) freigelegt. Mit geschlossenem Hahn oder Ventil 52 wird bei schwacher Drehung (zwischen 0 und 10 Umdrehungen pro Minute) und eingeschaltetem Hochfrequenzstrom die Resektion bzw. Ablation bis zur Obstruktion des distalen Endes des Rohrs 16 eingeleitet. Zu diesem Zeitpunkt wird der Hahn bzw. das Ventil 52 geöffnet und die Kombination des positiven Drucks der Spülung mit dem negativen Vakuum-Ansaugdruck über den Hahn bzw. Ventil 52 sowie eine Drehung der Schlinge 12 und der Wendel 14 bei mittlerer Geschwindigkeit mit eingeschaltetem Schnitt- oder Mischstrom ermöglichen eine kontinuierliche Resektion bspw. eines Myoms, wobei die drei Funktionen der Resektion, Abführung abgetrennten Gewebes und Koagulation gewährleistet werden.

Ein weiterer Vorteil der sich in das Rohr 16 erstreckenden Wendel 14 besteht darin, dass, sobald Gewebe in das Rohr 16 eingezogen wurde, auf Grund des sich in das Rohr 16 erstreckenden Abschnitts der Wendel 14 an dem Gewebe, bspw. einem Myom, gezogen werden kann, wodurch es zum Cavum angezogen werden kann, wodurch der bei einer herkömmlichen Resektion nicht zugängliche Zwischenteil des Gewebes angezogen und folglich reseziert werden kann. Damit kann ebenfalls begonnen werden, das Myom oder Prostataadenom von der Wand zu lösen, um sich eine bessere Übersicht von der Schnittstelle zu verschaffen und erheblich sicherer zu resezieren.

## Patentansprüche

1. Vorrichtung zur Resektion und/oder Ablation von organischem Gewebe mittels Hochfrequenzstrom, mit zumindest einer Wendel (14), wobei die Wendel (14) um die Längsachse der Wendel (14) in Rotation versetzbar ist, **dadurch gekennzeichnet, dass** distal von der zumindest einen Wendel (14) eine Schlinge (12) angeordnet ist, wobei die Wendel (14) und die Schlinge (12) mit Hochfrequenzspannung beaufschlagbar sind, und dass die Anordnung aus Schlinge (12) und Wendel (14) um die Längsachse der Wendel (14) in Rotation versetzbar ist.

2. Vorrichtung nach Anspruch 1, **dadurch gekennzeichnet, dass** die Wendel (14) als Doppelwendel oder Mehrfachwendel ausgebildet ist.

3. Vorrichtung nach Anspruch 1 oder 2, **dadurch gekennzeichnet, dass** die Wendel (14) in ihrem distalen Abschnitt (20) so ausgebildet ist, dass sie eine Abtragung seitlich von der Wendel (14) befindlichen Gewebes ermöglicht.

4. Vorrichtung nach einem der Ansprüche 1 bis 3, **dadurch gekennzeichnet, dass** die Wendel (14) so ausgebildet ist, dass sie einen Transport abgetrennten Gewebes nach proximal ermöglicht.

5. Vorrichtung nach einem der Ansprüche 1 bis 4, **dadurch gekennzeichnet, dass** die Wendel (14) teilweise von einem Rohr (16) umgeben und mit diesem zumindest teilweise fest verbunden ist, wobei ein distaler Abschnitt (20) der Wendel (14) aus einer distalen Öffnung (22) des Rohrs (16) aus dem Rohr (16) herausragt.

6. Vorrichtung nach Anspruch 5, **dadurch gekennzeichnet, dass** das Rohr (16) mittels eines Motors (30) in Rotation versetzbar ist.

7. Vorrichtung nach einem der Ansprüche 1 bis 4, **dadurch gekennzeichnet, dass** die Wendel (14) teilweise von einem Rohr (16) umgeben ist, und dass die Wendel (14) relativ zum Rohr (16) frei drehbar ist, wobei ein distaler Abschnitt (20) der Wendel (14) aus einer distalen Öffnung (22) des Rohrs (16) aus dem Rohr (16) herausragt.

8. Vorrichtung nach einem der Ansprüche 5 bis 7, **dadurch gekennzeichnet, dass** sich die Wendel (14) zumindest annähernd über die gesamte Länge des Rohrs (16) erstreckt.

9. Vorrichtung nach einem der Ansprüche 5 bis 7, **dadurch gekennzeichnet, dass** sich die Wendel (14) nur über eine Teillänge des Rohrs (16) erstreckt.

10. Vorrichtung nach einem der Ansprüche 5 bis 9, **dadurch gekennzeichnet, dass** der sich im Rohr (16) erstreckende Abschnitt der Wendel (14) elektrisch isoliert ist.

11. Vorrichtung nach einem der Ansprüche 5 bis 10, **dadurch gekennzeichnet, dass** das Rohr (16) elektrisch isoliert ist.

12. Vorrichtung nach einem der Ansprüche 5 bis 11, **dadurch gekennzeichnet, dass** der aus dem Rohr (16) herausragende Abschnitt (20) der Wendel (14) nicht elektrisch isoliert ist.

13. Vorrichtung nach einem der Ansprüche 5 bis 12, **dadurch gekennzeichnet, dass** der aus dem Rohr (16) herausragende Abschnitt (20) der Wendel (14) aus einem steifen Draht gebildet oder als Schneidklinge mit nach innen gerichteter Schneide ausgebildet ist.

14. Vorrichtung nach einem der Ansprüche 5 bis 13, **dadurch gekennzeichnet, dass** der sich im Rohr (16) erstreckende Abschnitt der Wendel (14) eine nach innen gerichtete Schneide aufweist.

15. Vorrichtung nach einem der Ansprüche 5 bis 14, **dadurch gekennzeichnet, dass** die Öffnung (22) des Rohrs (16) einen stumpfen oder schneidenden Rand aufweist.

16. Vorrichtung nach einem der Ansprüche 5 bis 15, **dadurch gekennzeichnet, dass** das Rohr (16) einen Durchmesser im Bereich von etwa 3 bis etwa 6,5 mm aufweist.

17. Vorrichtung nach einem der Ansprüche 1 bis 16, **dadurch gekennzeichnet, dass** die Schlinge (12) halbkreisförmig ausgebildet ist.

18. Vorrichtung nach einem der Ansprüche 1 bis 17, **dadurch gekennzeichnet, dass** die Schlinge (12) aus einem steifen elektrisch leitenden Draht gebildet oder als Schneidklinge ausgebildet ist, wobei im letzteren Fall die Schneide nach innen gerichtet ist.

19. Vorrichtung nach einem der Ansprüche 1 bis 18, **dadurch gekennzeichnet, dass** eine Ebene der Schlinge (12) gegenüber der Längsachse (24) geneigt, vorzugsweise geringfügig geneigt ist, derart, dass von der Schlinge (12) abgetrenntes Gewebe zum Inneren der Wendel (14) hin orientiert wird.

20. Vorrichtung nach einem der Ansprüche 1 bis 19, **dadurch gekennzeichnet, dass** die Drehgeschwindigkeit der Wendel (14) und der Schlinge (12) in einem Bereich von 0 bis etwa 1000 Umdrehungen pro Minute, vorzugsweise stufenlos, einstellbar ist.

21. Vorrichtung nach Anspruch 20, **dadurch gekennzeichnet, dass** zur Einstellung bzw. Steuerung der Drehgeschwindigkeit ein an einem proximal an der Vorrichtung vorhandenen Handgriff (46) angeordneter Betätigungsknopf (50) oder ein Fußpedal vorgesehen ist.

22. Vorrichtung nach einem der Ansprüche 1 bis 21, **dadurch gekennzeichnet, dass** der Hochfrequenzstrom von einem Hochfrequenzstromgenerator bereitgestellt wird und zum Schneiden und/oder Koagulieren ausgelegt ist, wobei der Hochfrequenzstrom, vorzugsweise über ein Fußpedal, einschaltbar ist.

23. Vorrichtung nach einem der Ansprüche 1 bis 22, **dadurch gekennzeichnet, dass** ein proximales Ende des Innenraums der Wendel (14) mit einem Auffangbehälter (40) zur Aufnahme von Gewebestücken verbunden ist.

24. Vorrichtung nach einem der Ansprüche 1 bis 23, **dadurch gekennzeichnet, dass** an den Innenraum der Wendel (14) ein vorzugsweise einstellbarer Unterdruck anlegbar ist.

25. Vorrichtung nach Anspruch 5 oder 7 und einem der Ansprüche 6, 8 bis 24, **dadurch gekennzeichnet, dass** ein Außenschaft (44) vorgesehen ist, um die Anordnung aus dem Rohr (16) und der Wendel (14) und der Schlinge (12) in dem Außenschaft (44) aufzunehmen, wobei die Schlinge (12) und der aus dem Rohr (16) herausragende Abschnitt (20) der Wendel (14) aus dem Außenschaft (44) herausragen.

26. Vorrichtung nach Anspruch 25, **dadurch gekennzeichnet, dass** der Außenschaft (44) zusätzlich zur Aufnahme eines Endoskops (56) ausgelegt ist.

27. Vorrichtung nach Anspruch 25 oder 26, **dadurch gekennzeichnet, dass** der Außenschaft (44) zumindest eine Leitung zum Zuführen von Spülflüssigkeit in das Operationsgebiet und/oder zumindest eine Leitung zum Absaugen von Flüssigkeit aus dem Operationsgebiet aufweist.

## Claims

1. A device for resection and/or ablation of organic tissue by means of a high-frequency current, comprising at least one helix (14), wherein the helix (14) is able to be moved in rotation about the longitudinal axis of the helix (14), **characterized in that** a loop (12) is arranged distally from the helix (14), wherein the helix (14) and the loop (12) can be acted upon by high-frequency voltage, and **in that** the arrangement of the loop (12) and the helix (14) is able to be moved in rotation about the longitudinal axis of the helix (14).

2. The device of claim 1, **characterized in that** the helix (14) is designed as a double helix or multiple helix.

3. The device of claim 1 or 2, **characterized in that** the helix (14), in its distal portion (20), is designed such that it permits ablation of tissue located laterally with respect to the helix (14).

4. The device of any one of claims 1 through 3, **characterized in that** the helix (14) is designed such that it permits transport of detached tissue in the proximal direction.

5. The device of any one of claims 1 through 4, **characterized in that** the helix (14) is partially surrounded by a tube (16) and is fixedly connected to the latter at least partially, and a distal portion (20) of the helix (14) protrudes from the tube (16) by way of a distal opening (22) of the tube (16).

6. The device of claim 5, **characterized in that** the tube (16) can be moved in rotation by means of a motor (30).

7. The device of any one of claims 1 through 4, **characterized in that** the helix (14) is partially surrounded by a tube (16), and **in that** the helix (14) is freely rotatable relative to the tube (16), and a distal portion (20) of the helix (14) protrudes from the tube (16) by way of a distal opening (22) of the tube (16).

8. The device of any one of claims 5 through 7, **characterized in that** the helix (14) extends at least approximately along the full length of the tube (16).

9. The device of any one of claims 5 through 7, **characterized in that** the helix (14) extends only along a partial length of the tube (16).

10. The device of any one of claims 5 through 9, **characterized in that** the portion of the helix (14) extending within the tube (16) is electrically insulated.

11. The device of any one of claims 5 through 10, **characterized in that** the tube (16) is electrically insulated.

12. The device of any one of claims 5 through 11, **characterized in that** the portion (20) of the helix (14) protruding from the tube (16) is not electrically insulated.

13. The device of any one of claims 5 through 12, **characterized in that** the portion (20) of the helix (14) protruding from the tube (16) is formed from a stiff wire or is designed as a cutting blade with inwardly directed cutting edge.

14. The device of any one of claims 5 through 13, **characterized in that** the portion of the helix (14) extending within the tube (16) has an inwardly directed cutting edge.

15. The device of any one of claims 5 through 14, **characterized in that** the opening (22) of the tube (16) has a blunt or sharp edge.

16. The device of any one of claims 5 through 15, **characterized in that** the tube (16) has a diameter in the range from approximately 3 to approximately 6.5 mm.

17. The device of any one of claims 1 through 16, **characterized in that** the loop (12) has a semicircular shape.

18. The device of any one of claims 1 through 17, **characterized in that** the loop (12) is formed from a stiff, electrically conductive wire or is designed as a cutting blade, and, in the latter case, the cutting edge is directed inward.

19. The device of any one of claims 1 through 18, **characterized in that** a plane of the loop (12) is inclined relative to the longitudinal axis (24), preferably slightly inclined, in such a way that tissue detached by the loop (12) is oriented toward the interior of the helix (14).

20. The device of any one of claims 1 through 19, **characterized in that** the speed of rotation of the helix (14) and of the loop (12) can be adjusted, preferably in a stepless manner, in a range from 0 to approximately 1000 revolutions per minute.

21. The device of claim 20, **characterized in that**, in order to adjust or control the speed of rotation, an actuating button (50) arranged on a handle (46) at the proximal end of the device is provided, or a foot pedal is provided.

22. The device of any one of claims 1 through 21, **characterized in that** the high-frequency current is supplied by a high-frequency current generator and is configured for cutting and/or coagulation, the high-frequency current preferably being able to be switched on via a foot pedal.

23. The device of any one of claims 1 through 22, **characterized in that** a proximal end of the interior of the helix (14) is connected to a collecting container (40) for receiving tissue fragments.

24. The device of any one of claims 1 through 23, **characterized in that** a preferably adjustable underpressure can be applied to the interior of the helix (14).

25. The device of claim 5 or 7 and of any one of claims 6, 8 through 24, **characterized in that** an outer shaft (44) is provided in order that the arrangement of tube (16), helix (14) and loop (12) can be received in the outer shaft (44), in which case the loop (12) and the portion (20) of the helix (14) protruding from the tube (16) protrude from the outer shaft (44).

26. The device of claim 25, **characterized in that** the outer shaft (44) is additionally designed to receive an endoscope (56).

27. The device of claim 25 or 26, **characterized in that** the outer shaft (44) comprises at least one line for delivering irrigation fluid to the operating site and/or at least one line for suctioning fluid away from the operating site.

## Revendications

1. Dispositif de résection et/ou d'ablation de tissu organique au moyen d'un courant haute fréquence, comportant au moins une spirale (14), la spirale (14) pouvant être amenée en rotation autour de l'axe longitudinal de la spirale (14), **caractérisé en ce qu'**une boucle (12) est disposée de manière distale par rapport à la au moins une spirale (14), la spirale (14) et la boucle (12) étant soumises à l'action d'une tension haute fréquence, et **en ce que** l'ensemble constitué de la boucle (12) et de la spirale (14) peut être amené en rotation autour de l'axe longitudinal de la spirale (14).

2. Dispositif selon la revendication 1, **caractérisé en ce que** la spirale (14) est réalisée comme une double spirale ou une spirale multiple.

3. Dispositif selon la revendication 1 ou 2, **caractérisé en ce que** la spirale (14) est réalisée de telle manière dans sa section distale (20) qu'elle permet d'enlever du tissu se trouvant sur le côté de la spirale (14).

4. Dispositif selon l'une quelconque des revendications 1 à 3, **caractérisé en ce que** la spirale (14) est réalisée de telle manière qu'elle permet un transport du tissu séparé vers le côté proximal.

5. Dispositif selon l'une quelconque des revendications 1 à 4, **caractérisé en ce que** la spirale (14) est entourée en partie par un tube (16) et est reliée au moins en partie de manière solide à ce dernier, une section (20) distale de la spirale (14) faisant saillie du tube (16) par un orifice (22) distal du tube (16).

6. Dispositif selon la revendication 5, **caractérisé en ce que** le tube (16) peut être amené en rotation au moyen d'un moteur (30).

7. Dispositif selon l'une quelconque des revendications 1 à 4, **caractérisé en ce que** la spirale (14) est entourée en partie par un tube (16), et **en ce que** la spirale (14) peut être tournée librement par rapport au tube (16), une section distale (20) de la spirale (14) faisant saillie du tube (16) par un orifice (22) distal du tube (16).

8. Dispositif selon l'une quelconque des revendications 5 à 7, **caractérisé en ce que** la spirale (14) s'étend au moins approximativement sur toute la longueur du tube (16).

9. Dispositif selon l'une quelconque des revendications 5 à 7, **caractérisé en ce que** la spirale (14) s'étend uniquement sur une longueur partielle du tube (16).

10. Dispositif selon l'une quelconque des revendications 5 à 9, **caractérisé en ce que** la section de la spirale (14) s'étendant dans le tube (16) est isolée électriquement.

11. Dispositif selon l'une quelconque des revendications 5 à 10, **caractérisé en ce que** le tube (16) est isolé électriquement.

12. Dispositif selon l'une quelconque des revendications 5 à 11, **caractérisé en ce que** la section (20) de la spirale (14) faisant saillie du tube (16) n'est pas isolée électriquement.

13. Dispositif selon l'une quelconque des revendications 5 à 12, **caractérisé en ce que** la section (20) de la spirale (14) faisant saillie du tube (16) est formée à partir d'un fil rigide ou est réalisée comme une lame tranchante dotée d'un tranchant orienté vers l'intérieur.

14. Dispositif selon l'une quelconque des revendications 5 à 13, **caractérisé en ce que** la section de la spirale (14) s'étendant dans le tube (16) présente un tranchant orienté vers l'intérieur.

15. Dispositif selon l'une quelconque des revendications 5 à 14, **caractérisé en ce que** l'orifice (22) du tube (16) présente un bord émoussé ou tranchant.

16. Dispositif selon l'une quelconque des revendications 5 à 15, **caractérisé en ce que** le tube (16) présente un diamètre se situant dans la plage allant d'environ 3 à environ 6,5 mm.

17. Dispositif selon l'une quelconque des revendications 1 à 16, **caractérisé en ce que** la boucle (12) est réalisée de manière à présenter une forme semi circulaire.

18. Dispositif selon l'une quelconque des revendications 1 à 17, **caractérisé en ce que** la boucle (12) est formée à partir d'un fil rigide électroconducteur ou est réalisée comme une lame tranchante, dans ce cas, le tranchant étant orienté vers l'intérieur.

19. Dispositif selon l'une quelconque des revendications 1 à 18, **caractérisé en ce qu'**un plan de la boucle (12) est incliné, de préférence légèrement incliné, par rapport à l'axe longitudinal (24) de telle manière que le tissu séparé par la boucle (12) est orienté en direction de l'intérieur de la spirale (14).

20. Dispositif selon l'une quelconque des revendications 1 à 19, **caractérisé en ce que** la vitesse de rotation de la spirale (14) et de la boucle (12) peut être réglée dans une plage allant de 0 jusqu'à environ 1000 rotations par minute, de préférence sans palier.

21. Dispositif selon la revendication 20, **caractérisé en ce qu'**un bouton d'actionnement (50) disposé sur une poignée (46) présente de manière proximale sur le dispositif ou une pédale sont prévus pour le réglage ou la commande de la vitesse de rotation.

22. Dispositif selon l'une quelconque des revendications 1 à 21, **caractérisé en ce que** le courant haute fréquence est fourni par un générateur de courant haute fréquence et est configuré pour la découpe et/ou la coagulation, le courant haute fréquence pouvant mis en service, de préférence par l'intermédiaire d'une pédale.

23. Dispositif selon l'une quelconque des revendications 1 à 22, **caractérisé en ce qu'**une extrémité proximale de l'espace intérieur de la spirale (14) est reliée à un récipient collecteur (40) servant à recevoir des fragments de tissu.

24. Dispositif selon l'une quelconque des revendications 1 à 23, **caractérisé en ce qu'**une dépression de préférence ajustable peut être appliquée au niveau de l'espace intérieur de la spirale (14).

25. Dispositif selon la revendication 5 ou 7 et selon l'une quelconque des revendications 6, 8 à 24, **caractérisé en ce qu'**une tige extérieure (44) est prévue pour recevoir l'ensemble constitué du tube (16) et de la spirale (14) et de la boucle (12) dans la tige extérieure (44), la boucle (12) et la section (20) de la spirale (14) faisant saillie du tube (16) faisant saillie de la tige extérieure (44).

26. Dispositif selon la revendication 25, **caractérisé en ce que** la tige extérieure (44) est configurée en complément pour recevoir un endoscope (56).

27. Dispositif selon la revendication 25 ou 26, **caractérisé en ce que** la tige extérieure (44) présente au moins une conduite servant à amener du liquide de rinçage dans la zone opératoire et/ou au moins une conduite servant à aspirer le liquide hors de la zone opératoire.
